(19) Europäisches Patentamt
European Patent Office
Office européen des brevets

(11) Veröffentlichungsnummer: **0 138 018**
**B1**

(12) # EUROPÄISCHE PATENTSCHRIFT

(45) Veröffentlichungstag der Patentschrift: **02.11.88**

(51) Int. Cl.[4]: **A 61 K 47/00**, A 61 K 31/495, A 61 K 31/535

(21) Anmeldenummer: **84110474.8**

(22) Anmeldetag: **04.09.84**

(54) **Lösungen milchsaurer Salze von Piperazinylchinolon- und Piperazinyl-azachinoloncarbonsäuren.**

(30) Priorität: **17.09.83 DE 3333719**

(43) Veröffentlichungstag der Anmeldung: **24.04.85 Patentblatt 85/17**

(45) Bekanntmachung des Hinweises auf die Patenterteilung: **02.11.88 Patentblatt 88/44**

(84) Benannte Vertragsstaaten: **AT BE CH DE FR GB IT LI NL SE**

(56) Entgegenhaltungen:
**EP-A-0 009 425**
**CH-A-296 839**
**DE-A-2 918 319**
**US-A-4 022 894**

**CHEMICAL ABSTRACTS, Band 93, Nr. 20, November 1980, Seite 359, Nr. 192029f, Columbus, Ohio, US; & JP - A - 80 31 028 (DAIICHI SEIYAKU CO., LTD.) 05.03.1980**
**CHEMICAL ABSTRACTS, Band 95, Nr. 24, Dezember 1981, Seite 370, Nr. 209557t, Columbus, Ohio, US; VAN DER MEER, Y.G. et al.: "Formulation and stability of a trimethoprim injection" & PHARM. WEEKBL. 1981, 116(40), 1211-13**
**CHEMICAL ABSTRACTS, Band 98, Nr. 8, Februar 1983, Seite 357, Nr. 59941j, Columbus, Ohio, US; & JP - A - 57 188 515 (KYOTO PHARMACEUTICAL INDUSTRIES, LTD.) 19.11.1982**

(73) Patentinhaber: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**
Erfinder: **Lammens, Robert, Dr., Walter- Flex-Strasse 30 e, D-5090 Leverkusen 1 (DE)**

**EP 0 138 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft Lösungen von Piperazinyl-chinolon-und Piperazinyl-azachinoloncarbonsäuren und zwar sowohl gebrauchsfertige Injektions- und/oder Infusionslösungen als auch Lösungen, die vor der Applikation in solche Injektions und/oder Infusionslösungen verführt werden können.

Die erfindungsgemäßen Lösungen sind dadurch gekennzeichnet, daß sie außer dem Milchsäuresalz der aktiven Substanz und gegebenenfalls üblichen Hilfsstoffen zusätzlich mindestens eine nicht zu Fällungen führende Säure, insbesondere Milchsäure, enthalten.

Als aktive Substanzen kommen in Frage mindestens eine der Verbindungen der allgemeinen Formeln I oder II

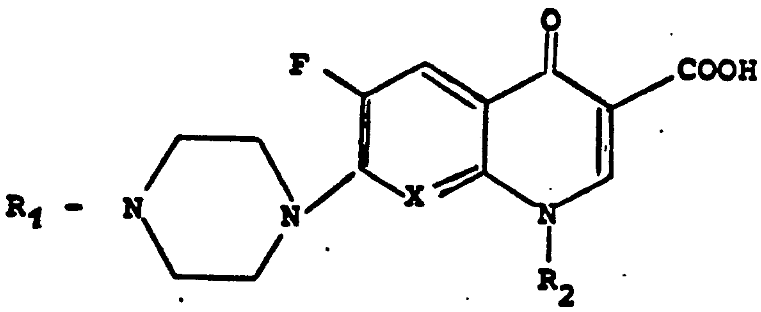

(I)

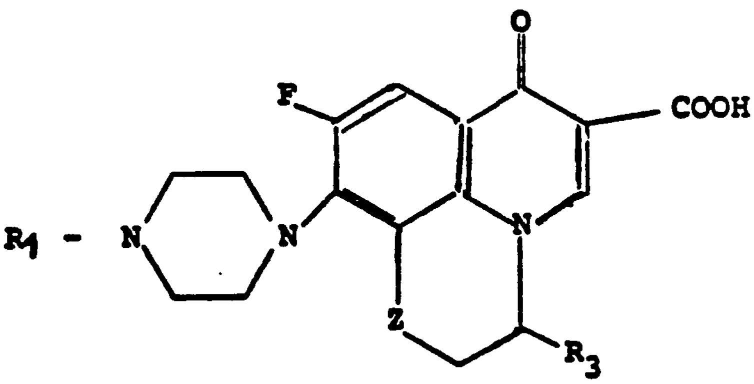

(II)

in denen

X = N, C-H oder C-F,
Z = O oder $CH_2$,
$R_1$ = Wasserstoff, Methyl, Ethyl oder β-Hydroxyethyl,
$R_2$ = Cyclopropyl oder Ethyl und
$R_3$ = Wasserstoff, Methyl oder Ethyl bedeutet.

Insbesondere seien genannt:
- 1-Cyclopropyl-6-fluor-1,4-dihydro-4-oxo-7-(1-piperazinyl)-chinolin-3-carbonsäure (Verbindung A);
- Ethyl-6-fluor-1,4-dihydro-4-oxo-7-(1-piperazinyl)-chinolin-3-carbonsäure (Verbindung B);
- 9-Fluor-3-methyl-10-(4-methyl-1-piperazinyl)-7-oxo-2,3-dihydro-7H-pyrido[1,2,3-de]1,4-benzoxazin-6-carbonsäure (Verbindung C);
- 1-Ethyl-6-fluor-1,4-dihydro-4-oxo-7-(1-piperazinyl)-1,8-naphthyridin-3-carbonsäure (Verbindung D);
- 1,Cyclopropyl-6-fluor-1,4-dihydro-4-oxo-7-(4-ethyl-1-piperazinyl)-chinolin-3-carbonsäure

Diese Substanzen haben bekanntlich hohe antibakterielle Wirkungen und eignen sich daher als Arzneimittel zur Bekämpfung von bakteriellen Infektionen bei Menschen und Tieren.

Die Verbindungen der Formel I sind bekannt aus:
- J. Med. Chem. 23 (1980) 1358; DE-OS-3 142 854; DE-OS-3 033 157; EP-OS-0 067 666.

Die Verbindungen der Formel II sind bekannt aus:
- EP-OS-0 047 005; DE-OS-3 037 103; DE-OS-2 914 258.

Als übliche Hilfsstoffe für die erfindungsgemäßen Lösungen kommen nicht-toxische, pharmazeutische Stoffe in Frage. Dies sind zum Beispiel Verdickungsmittel, Resorptionsbeschleuniger, Resorptionshemmer, Kristallisationsverzögerer, Komplexierungsmittel, Antioxidantia, Isotonisierungsmittel oder Dehydrierungsmittel. Sie können feste, halbfeste oder flüssige Konsistenz haben.

Als nicht zu Fällungen führende Säuren kommen zum Beispiel in Frage Methansulfonsäure, Propionsäure, Bernsteinsäure, Salzsäure, insbesondere aber Milchsäure.

Aus unterschiedlichen Gründen werden in der Pharmazie öfters Säureadditionssalze oder Alkalisalze von Wirkstoffen eingesetzt. Additionssalze der aktiven Substanzen können aus mehreren anorganischen und organischen Säuren, wie z. B. Schwefelsäure, Salpetersäure, Salzsäure, Citronensäure, Essigsäure, Äpfelsäure, Bernsteinsäure, Weinsäure, Fumarsäure und Methansulfonsäure hergestellt werden.

Viele solcher Salze sind jedoch nicht oder sehr wenig geeignet zum Herstellen von Infusions- und/oder Injektionslösungen, weil z. B. der pH und/oder die Löslichkeit und/oder die Haltbarkeit, insbesondere bezüglich Ausscheidungen, der gebrauchsfertigen Infusions- und/oder Injektionslösung nicht den an solchen Lösungen zu stellenden pharmazeutischen Anforderungen entsprechen.

Es wurde nun gefunden, daß die Lösungen haltbar sind, wenn sie außer dem Milchsäuresalz von mindestens einer der aktiven Substanzen und gegebenenfalls üblichen Hilfsstoffen, zusätzlich mindestens eine nicht zu Fällungen führende Säure, insbesondere Milchsäure, enthalten.

Die Anwesenheit solcher Säuren, insbesondere der Milchsäure, oder je nach pH ein Gemisch von Säure(n) und deren Anion(en) ist wesentlich für die Stabilität der zu applizierenden Lösung, insbesondere bezüglich Ausscheidungen.

Je nach verwendeten Primärpackmitteln, der Konzentration der aktiven Substanz in der Lösung, dem pH der Lösung und den gestellten Haltbarkeitsanforderungen kann der Milchsäuregehalt der erfindungsgemäßen Lösungen 0,1 bis 90 % betragen. Der Milchsäuregehalt der zu applizierenden Lösung kann 0,1 bis 10 % betragen, vorzugsweise 0,5 bis 1,4 %.

Diese Mengenangaben beziehen sich auf die gesamte Menge Säure, d. h. undissoziierte und dissoziierte Säure.

Wenn im Nachstehenden von diesen für die Stabilität erforderlichen Säuren, insbesondere Milchsäure die Rede ist, dann wird damit die gesamte Menge Säure gemeint, d. h. undissoziierte und dissoziierte Säure.

Bei der Verwendung von anderen nicht zu Fällungen führenden Säuren, wie z. B. Methansulfonsäure, Propionsäure, Salzsäure oder Bernsteinsäure, kann der Säuregehalt je nach Konzentration der aktiven Substanz, den gestellten Haltbarkeitsanforderungen und dem pH der gebrauchsfertigen erfindungsgemäßen Lösungen 0,05 bis 4 % betragen, vorzugsweise zwischen 0,3 und 2 %.

Der pH der gebrauchsfertigen erfindungsgemäßen Lösungen kann zwischen 2,5 und 7 liegen, vorzugsweise zwischen 3,5 und 4,5.

Es war sehr überraschend zu finden, daß durch den Zusatz von mindestens einer nicht zu Fällungen führenden Säure, insbesondere der Milchsäure, in Mengen wie oben angegeben auch Infusions- und/oder Injektionslösungen des Milchsäuresalzes von Verbindung B und D stabilisiert werden konnten, während die 1 : 1 stoechiometrischen Salze dieser Verbindung zu Ausscheidungen neigten. Dies trifft auch für die erfindungsgemäßen Zubereitungen der Verbindung C zu.

Weiterhin wurde gefunden, daß es mehrere Verfahrensmöglichkeiten zur Herstellung der erfindungsgemäßen Lösungen gibt.

Zur Herstellung der Lösung der aktiven Substanz kann man vom Milchsäuresalz der aktiven Substanz oder einem Hydrat desselben ausgehen. Dabei ist es möglich, den erforderlichen Zusatz oder einen Teil dieses Zusatzes von mindestens einer nicht zu Fällungen führenden Säure, insbesondere Milchsäure, in das Milchsäuresalz einzuarbeiten, z. B. durch Gefriergetrocknung.

Man kann aber auch die Milchsäuresalze direkt in der Lösung herstellen, und zwar durch Zugabe der zur Salzbildung erforderlichen Mengen Milchsäure.

In dieser Weise kann man sowohl gebrauchsfertige Lösungen der aktiven Substanz, abgefüllt in geeignete Behälter, zum Beispiel in Ampullen, Injektions- oder Infusionsflaschen, als auch zur Herstellung solcher Lösungen geeignete Vorprodukte, z. B. Konzentrate oder Trockenampullen, herstellen.

Die erfindungsgemäßen Lösungen sollen ebenso wie die ihnen zugrunde liegenden Verbindungen der Formeln I und II als Arzneimittel zur Bekämpfung bakterieller Infektionen verwendet werden. Als Anwendung kommen Injektionen und Infusionen in Frage. Die Dosierungen entsprechen denjenigen der bekannten Verbindungen A, B, C und D.

## Formulierungen

Die erfindungsgemäßen Lösungen werden hergestellt durch Lösen der aktiven Substanz oder deren Milchsäuresalz und gegebenenfalls üblicher Hilfsstoffe in einer Lösung von Milchsäure oder einem Gemisch aus Milchsäure und z. B. Natriumlaktat, gegebenenfalls unter leichter Erwärmung.

Eventuell wird noch Wasser oder ein Gemisch von Wasser und Natronlauge zum Einstellen der gewünschten Konzentration der aktiven Substanz und/oder des pH's der Lösung zugegeben.

**Beispiel 1**

| | |
|---|---|
| 1-Cyclopropyl-6-fluor-1,4-dihydro-4-oxo-7-(1-piperazinyl-chinolon-3-carbonsäure (Verbindung A) | - 150 g |
| Milchsäure (90 Gew.-%) | - 262 g |
| 2 m Natronlauge | - 266 g |
| Wasser | ad 15,0 Liter |

**Beispiel 2**

| | |
|---|---|
| Monolaktat von Verbindung A | - 1,27 g |
| Milchsäure (90 Gew.-%) | - 1,45 g |
| 2 m Natronlauge | - 1,80 g |
| Mannit | - 1,37 g |
| Wasser | ad 100,0 ml |

**Beispiel 3**

| | |
|---|---|
| Verbindung A | - 10,00 g |
| Milchsäure (90 Gew.-%) | - 4,85 g |
| Wasser | ad 1000,0 ml |

**Beispiel 4**

| | |
|---|---|
| 1-Ethyl-6-fluor-1,4-dihydro-4-oxo-7-(1-piperazinyl)-chinolon-3-carbonsäure (Verbindung B) | - 1,00 g |
| Milchsäure (90 Gew.-%) | - 0,50 g |
| Glukose | - 3,85 g |
| Wasser | ad 100,00 ml |

**Beispiel 5**

| | |
|---|---|
| 9-Fluor-3-methyl-10-(4-methyl-1-piperazinyl)-7-oxo-2,3-dihydro-7H-pyrido-[1,2,3-de]-4 benzoaxzin-6-carbonsäure (Verbindung C) | - 5,00 g |
| Milchsäure (90 Gew.-%) | - 2,20 g |
| Wasser | ad 100,00 ml |

**Beispiel 6**

| | |
|---|---|
| Dihydrat des Monolaktats von Verbindung A | - 69,0 g |
| Milchsäure (90 Gew.-%) | - 24,2 g |
| Wasser | ad 1000,0 ml |

**Beispiel 7**

| | |
|---|---|
| Verbindung A | - 300,0 g |
| Milchsäure (90 Gew.-%) | - 177,3 g |
| Wasser | ad 6000,0 ml |

**Beispiel 8**

| | |
|---|---|
| Verbindung A | - 200,0 g |
| Milchsäure (90 Gew.-%) | - 75,0 g |
| Wasser | ad 2000,0 ml |

**Beispiel 9**

| | |
|---|---|
| Monolaktat von Verbindung B | - 128,2 g |
| Milchsäure (90 Gew.-%) | - 50,0 g |
| Wasser | ad 1,000,0 ml |

**Beispiel 10**

| | |
|---|---|
| Verbindung C | - 100,0 g |
| Milchsäure (90 Gew.-%) | - 44,4 g |
| Wasser | ad 1000,0 ml |

**Beispiel 11**

| | |
|---|---|
| Verbindung A | - 30,0 g |
| Milchsäure (90 Gew.-%) | ad 100,0 g |

**Beispiel 12**

| | |
|---|---|
| Verbindung A | 1,00 g |
| Milchsäure (90 Gew.-%) | 0,30 g |
| Bernsteinsäure | 0,71 g |
| Wasser | ad 100,00 ml |

Mit 2 m Natronlauge auf pH 3,6 eingestellt.

**Beispiel 13**

| | |
|---|---|
| Monolaktat von Verbindung A | 1,27 g |
| Methansulfonsäure | 0,60 g |
| Wasser | ad 100,00 ml |

Mit 2 m Natronlauge auf pH 3,9 eingestellt.

**Beispiel 14**

| | |
|---|---|
| Monolaktat von Verbindung A | 1,27 g |
| Milchsäure (90 Gew.-%) | 0,56 g |
| Methansulfonsäure | 1,45 g |
| Wasser | ad 100,00 ml |

Mit 2 m Natronlauge auf pH 3,7 eingestellt.

**Beispiel 15**

| | |
|---|---|
| Verbindung A | 1,00 g |
| Milchsäure (90 Gew.-%) | 0,86 g |
| Propionsäure | 1,12 g |
| Wasser | ad 100,00 ml |

Mit 2 m Natronlauge auf pH 3,8 eingestellt.

**Beispiel 16**

| | |
|---|---|
| Verbindung B | 1,00 g |
| Milchsäure (90 Gew.-%) | 0,87 g |
| Propionsäure | 0,46 g |
| Wasser | ad 100,00 ml |

**Beispiel 17**

| | |
|---|---|
| Monolaktat von Verbindung B | 1,28 g |
| Milchsäure (90 Gew.-%) | 1,11 g |
| Bernsteinsäure | 1,85 g |
| Wasser | ad 100,00 ml |

Mit 2 m Natronlauge auf pH 3,7 eingestellt.

**Beispiel 18**

| | |
|---|---|
| Verbindung B | 1,00 g |
| Milchsäure (90 Gew.-%) | 0,42 g |
| Methansulfonsäure | 1,50 g |
| Wasser | ad 100,00 ml |

Mit 2 m Natronlauge auf pH 3,8 eingestellt.

**Beispiel 19**

| | |
|---|---|
| Verbindung C | 1,00 g |
| Milchsäure (90 Gew.-%) | 0,39 g |
| Methansulfonsäure | 0,53 g |
| Wasser | ad 100,00 ml |

Mit 2 m Natronlauge auf pH 3,9 eingestellt.

**Beispiel 20**

| | |
|---|---|
| Monolaktat von Verbindung C | 1,25 g |
| Milchsäure (90 Gew.-%) | 0,56 g |
| Propionsäure | 1,03 g |
| Wasser | ad 100,00 ml |

**Beispiel 21**

| | |
|---|---|
| Verbindung C | 1,00 g |
| Milchsäure (90 Gew.-%) | 1,39 g |
| Bernsteinsäure | 0,64 g |
| Wasser | ad 100,00 ml |

Mit 2 m Natronlauge auf pH 3,7 eingestellt

**Beispiel 22**

| | |
|---|---|
| Verbindung A | 1,00 g |
| Milchsäure (10 Gew.-%) | 0,33 g |
| 1 m Salzsäure | 1,20 g |
| Wasser | ad 100,00 ml |

**Beispiel 23**

| | |
|---|---|
| Verbindung A | 1,00 g |
| Milchsäure (90 Gew.-%) | 0,41 g |
| Wasser | ad 100,00 ml |

Mit 1 m Salzsäure auf pH 4,0 eingestellt.

**Patentansprüche**

1. Lösungen milchsaurer Salze von Piperazinyl-chinolon-und Piperazinyl-azachinolon-carbonsäuren der allgemeinen Formeln I oder II

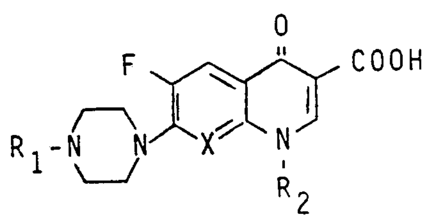

(I)

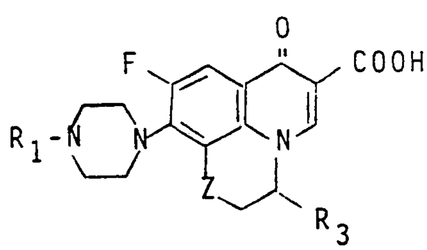

(II)

in denen

X = N, C-H oder C-F,

Z = O oder $CH_2$,
$R_1$ = Wasserstoff, Methyl, Ethyl, oder β-Hydroxyethyl,
$R_2$ = Cyclopropyl oder Ethyl und
$R_3$ = Wasserstoff, Methyl oder Ethyl bedeutet,

die außer den genannten milchsauren Salzen und gegebenenfalls üblichen Hilfsstoffen zusätzlich mindestens eine nicht zu Fällungen führende Säure enthalten.

2. Lösungen nach Anspruch 1, die als nicht zu Fällungen führende Säure Milchsäure enthalten.

3. Lösungen nach Anspruch 1, die als milchsaures Salz das Laktat der 1-Cyclopropyl-6-fluor-1,4-dihydro-4-oxo-7-(1-piperazinyl)-chinolin-3-carbonsäure enthalten.

4. Lösungen nach Anspruch 1, deren Milchsäuregehalt 0,1 bis 90 % beträgt.

5. Gebrauchsfertige Lösungen nach Anspruch 1, deren Milchsäuregehalt 0,1 bis 10 %, vorzugsweise 0,5 bis 1,4 % beträgt.

6. Verfahren zur Herstellung von Lösungen milchsaurer Salze von Piperazinyl-chinolon- und Piperazinyl-azachinolon-carbonsäuren der allgemeinen Formeln I oder II

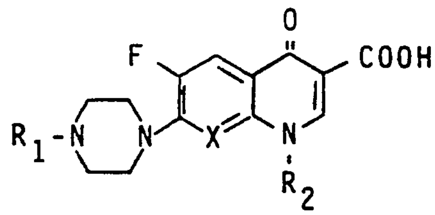

(I)

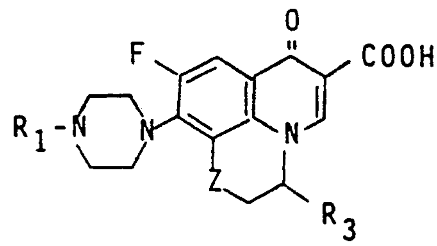

(II)

in denen

X = H, C-H oder C-F,
Z = O oder $CH_2$,
$R_1$ = Wasserstoff, Methyl, Ethyl, oder β-Hydroxyethyl,
$R_2$ = Cyclopropyl oder Ethyl und
$R_3$ = Wasserstoff, Methyl oder Ethyl bedeutet,

dadurch gekennzeichnet, daß man Lösungen von milchsauren Salzen der Säuren der Formeln (I) oder (II) mit 0,1 bis 90 % Milchsäure oder mit 0,1 bis 10 % mindestens einer nicht zur Fällung führenden Säure unter Erwärmen umsetzt.

7. Antibakterielles Arzneimittel, gekennzeichnet durch einen Gehalt an mindestens einer Lösung gemäß Anspruch 1.

**Revendications**

1. Solutions de sels lactiques d'acides pipérazinyl-quinolone- et d'acide pipérazinyl-azaquinolone-carboxyliques de formules générales I et II

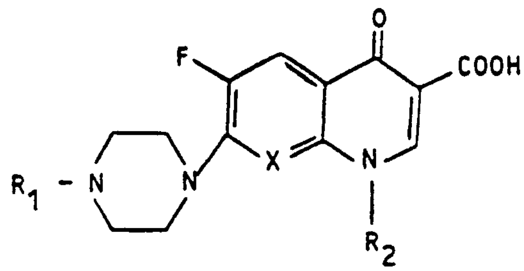

(I)

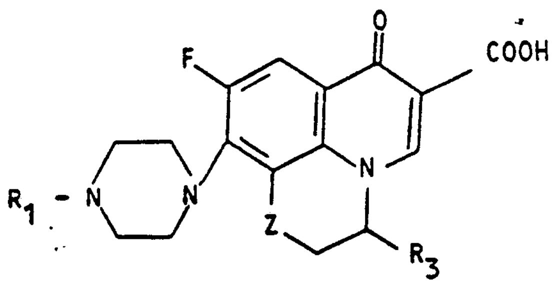

(II)

dans lesquelles

X représente N, C-H ou C-F,
Z représente O ou $CH_2$,
$R_1$ représente l'hydrogène ou un groupe méthyle, éthyle ou β-hydroxyéthyle,
$R_2$ représente un groupe cyclopropyle ou éthyle et
$R_3$ représente l'hydrogène ou un groupe méthyle ou éthyle,

qui, outre les sels lactiques cités et éventuellement des substances auxiliaire habituelles, contiennent en outre au moins un acide ne conduisant pas à des précipitations.

2. Solutions selon la revendication 1, qui contiennent de l'acide lactique comme acide ne conduisant pas à des précipitations.

3. Solutions selon la revendication 1, qui contiennent comme sel lactique le lactate de l'acide 1-cyclopropyl-6-fluoro-1,4-dihydro-4-oxo-7-(1-pipérazinyl)-quinoléine -3-carboxylique.

4. Solutions selon la revendication 1, dont la teneur en acide lactique est de 0,1 à 90 %.

5. Solutions prêtes à l'emploi selon la revendication 1, dont la teneur en acide lactique est de 0,1 à 10 %, de préférence de 0,5 à 1,4 %.

6. Procédé pour la fabrication de solutions de sels lactiques d'acides pipérazinyl-quinolone- et pipérazinyl-azaquinolone-carboxyliques de formules générales I et II

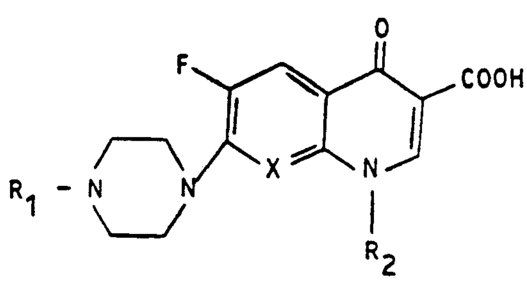

(I)

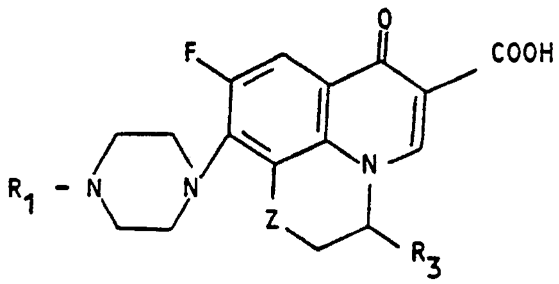

(II)

dans lesquelles

X représente N, C-H ou C-F,
Z représente O ou $CH_2$,
$R_1$ représente l'hydrogène ou un groupe méthyle, éthyle ou β-hydroxyéthyle,
$R_2$ représente un groupe cyclopropyle ou éthyle et
$R_3$ représente l'hydrogène ou un groupe méthyle ou éthyle,

caractérisé en ce que l'on fait réagir en chauffant des solutions de sels lactiques des acides de formules I ou II avec 0,1 à 90 % d'acide lactique ou avec 0,1 à 10 % d'au moins un acide ne conduisant pas à des précipitations.

7. Médicament antibactérien, caractérisé en ce qu'il contient au moins une solution selon la revendication 1.

## Claims

1. Solutions of lactic acid salts of piperazinyl-quinolone- and piperazinyl-azaquinolone-carboxylic acids of the general formulae I or II

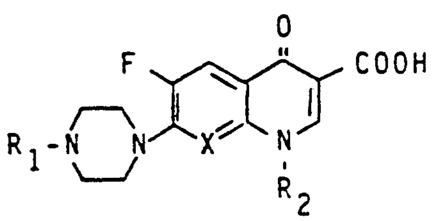

(I)

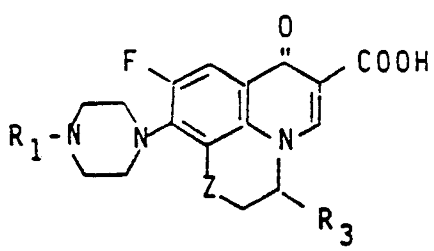

(II)

in which

X denotes N, C-H or C-F,
Z denotes O or $CH_2$,
$R_1$ denotes hydrogen, methyl, ethyl or β-hydroxyethyl,
$R_2$ denotes cyclopropyl or ethyl and
$R_3$ denotes hydrogen, methyl or ethyl,

which, besides the lactic acid salts mentioned and, if appropriate, customary auxiliaries, additionally contain at least one acid which does not lead to precipitates.

2. Solutions according to claim 1, which contain lactic acid as the acid which does not lead to precipitates.

3. Solutions according to claim 1, which contain the lactate of 1-cyclopropyl-6-fluoro-1,4-dihydro-4-oxo-7-(1-piperazinyl)-quinoline-3-carboxylic acid as the lactic acid salt.

4. Solutions according to claim 1, in which the tactic acid content is 0.1 to 90 %.

5. Ready-to-use solutions according to claim 1, in which the lactic acid content is 0.1 to 10 %, preferably 0.5 to 1.4 %.

6. Process for the preparation of solutions of lactic acid salts of piperazinyl-quinolone- and piperazinylazaquinolone-carboxylic acids of the general formulae I or II

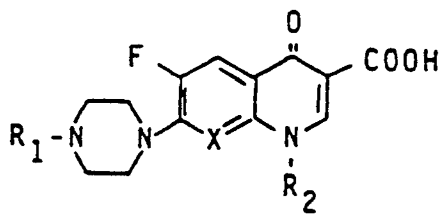

(I)

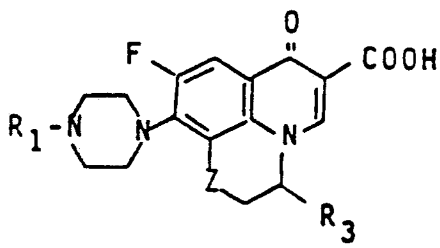

(II)

in which

| | |
|---|---|
| X | denotes N, C-H or C-F, |
| Z | denotes O or $CH_2$, |
| $R_1$ | denotes hydrogen, methyl, ethyl or β-hydroxyethyl, |
| $R_2$ | denotes cyclopropyl or ethyl and |
| $R_3$ | denotes hydrogen, methyl or ethyl, |

characterised in that solutions of lactic acid salts of the acids of the formulae (I) or (II) are reacted with 0.1 to 90 % of lactic acid or with 0.1 to 10 % of at least one acid which does not lead to precipitation, with warming.

7. Antibacterial medicaments, characterised in that they contain at least one solution according to claim 1.